# EUROPEAN PATENT APPLICATION

(11) **EP 4 094 832 A1**
(43) Date of publication of application: **30.11.2022**
(21) Application number: 21744915.6
(22) Date of filing: 20.01.2021
(51) Int. Cl.: B01J 35/02, A61L 9/00, A61L 9/18, A61L 9/20

(54) **PHOTOCATALYST UNIT AND MASK PROVIDED WITH PHOTOCATALYST UNIT**

(30) Priority: 24.01.2020 JP 2020009694; 18.12.2020 JP 2020210179
(71) Applicant: APS Japan Co., Ltd., Osaka-shi, Osaka 541-0059 (JP)
(72) Inventor: WATANABE, Teruo, Osaka-shi, Osaka 541-0059 (JP); WATANABE, Hidemitsu, Osaka-shi, Osaka 541-0059 (JP); WATANABE, Hiroyuki, Osaka-shi, Osaka 541-0059 (JP); WATANABE, Takafumi, Osaka-shi, Osaka 541-0059 (JP); YAMAGUCHI, Masao, Osaka-shi, Osaka 541-0059 (JP)
(74) Representative: Vossius & Partner Patentanwälte Rechtsanwälte mbB
(86) International application number: PCT/JP2021/001858
(87) International publication number: WO 2021/149724

(57) **Abstract**

Provided is a photocatalyst unit that can improve the fluid cleaning action using a photocatalyst by efficiently applying ultraviolet light to a photocatalyst, and at the same time, that can, as a photocatalyst unit including a photocatalyst filter and a light application part, be reduced in thickness and size, and that has a high degree of freedom in design of a flow path structure.

A photocatalyst carrier 3 is an impeller 4 including a plurality of vane plate parts 41 each of which has a surface carrying a photocatalyst, and the plurality of vane plate parts 41 rotate upon receiving a pressure from a fluid passing through a fluid passage path 10. A light application part 5 is provided so as to apply light to the rotating vane plate part 41 of the impeller 4, from a predetermined position on an outer circumferential side with respect to the vane plate parts 41 of the impeller 4.

## Description

### TECHNICAL FIELD

The present invention relates to a photocatalyst unit that cleans a fluid such as air by a photocatalyst, and relates to a mask provided with the photocatalyst unit.

### BACKGROUND ART

To date, air cleaners having air cleaning structures for decomposing viruses or the like by using photocatalysts have been proposed (e.g., see Patent Literatures 1, 2). These conventional air cleaning structures using photocatalysts are configured as follows. That is, one surface out of obverse and reverse surfaces of a plate-shaped base member (filter base member) having a large number of air passage holes penetrating in the thickness direction thereof carries a photocatalyst such as a titanium oxide, to form a photocatalyst filter. Then, a photocatalyst unit in which a plurality of ultraviolet lamps are disposed with an interval therebetween at positions opposed to the photocatalyst carrying surface of the photocatalyst filter is provided. To this unit, air is supplied in the filter thickness direction from one side out of the obverse and reverse sides, thereby causing air to circulate through the air passage holes of the filter, the photocatalyst layer, and gaps between the plurality of ultraviolet lamps. Then, in the course of passing the photocatalyst layer, hazardous substances in the air are decomposed/removed by the photocatalyst excited by ultraviolet light.

In such a conventional air cleaning structure using a photocatalyst unit, the photocatalyst filter and the ultraviolet lamps are disposed so as to face each other in the thickness direction of the filter. This increases the thickness of the unit and provides a limitation in making the unit thin and compact. In addition, due to the structure in which air circulates between the ultraviolet lamps, there is also a limitation in increasing the number of ultraviolet lamps that cause resistance to air, and there is also a certain limitation in improving the application amount and application efficiency of ultraviolet light to the photocatalyst filter. For example, even when a photocatalyst is carried to the depth at the inner surface of the air passage holes of the plate-shaped base member, it is difficult to efficiently apply ultraviolet light to the depth.

In contrast to this, the following air cleaning structure has been proposed (see Patent Literature 3). That is, in the air cleaning structure, a plurality of plate-shaped portions each having obverse and reverse surfaces each carrying a photocatalyst are disposed such that the obverse surface and the reverse surface of plate-shaped portions adjacent to each other are opposed to each other with a gap therebetween, to form a photocatalyst filter having air circulation paths defined by the gaps. An ultraviolet application unit that applies ultraviolet light toward the gaps is disposed at a position that is opposed to, among end surfaces of each plate-shaped portion of the photocatalyst filter, an end surface on at least one side out of an end surface on the air entrance side to the gap and an end surface on the air exit side from the gap, the position being separated from the end surface by a predetermined distance. Between the ultraviolet application unit and the end surface of each plate-shaped portion, an air supply path which takes in air from a lateral direction substantially parallel to the end surface and supplies air to the circulation path defined by the gap between the plate-shaped portions, or an air discharge path which discharges air exiting from the circulation path to a lateral direction substantially parallel to the end surface, are formed.

In such an air cleaning structure, the ultraviolet application unit is disposed at a position separated, by a predetermined distance, from the end surface on the air entrance side or air exit side, out of the end surfaces of each plate-shaped portion of the photocatalyst filter, and the air supply path which takes in air from a lateral direction substantially parallel to the end surface, or the air discharge path which discharges air to a lateral direction substantially parallel to the end surface, are formed between the ultraviolet application unit and the end surface of each plate-shaped portion. Thus, in this structure, air is not supplied or discharged through gaps between ultraviolet lamps, so that the degree of freedom of design of the ultraviolet application unit is significantly improved, and ultraviolet light can be more efficiently applied to the photocatalyst at each plate-shaped portion, whereby significant improvement of the air cleaning action by the photocatalyst is facilitated.

However, in this air cleaning structure, air is supplied or discharged in an L-shape from a lateral direction between the ultraviolet application unit and the photocatalyst filter. Therefore, the form (flow path) of supply/discharge of air is limited, and the entirety of the unit is inevitably increased in size. In addition, in a case where a fluid is to be caused to flow straight in the filter thickness direction, not in an L-shape, there is a problem that the aforementioned configuration cannot be adopted and the flow path structure is limited.

### CITATION LIST

### [PATENT LITERATURE]

[PTL 1] Utility Model Registration No. 3150894
[PTL 2] Japanese Unexamined Patent Application Publication (Translation of PCT Application) No. 2011/114894
[PTL 3] Japanese Unexamined Patent Application Publication No. 2017-148484

### SUMMARY OF THE INVENTION

### PROBLEMS TO BE SOLVED BY THE INVENTION

Thus, in consideration of the situation described above, an object of the present invention is to provide a photocatalyst unit that can improve the fluid cleaning action by a photocatalyst by efficiently applying ultraviolet light to the photocatalyst, and at the same time, that can, as a photocatalyst unit including a photocatalyst filter and a light application part, be reduced in thickness and size, and that has a high degree of freedom in design of the flow path structure, and a mask provided with the photocatalyst unit.

### SOLUTION TO THE PROBLEMS

The present applicant has already proposed the following fluid cleaning structure, using a photocatalyst, which can be reduced in thickness and size, and has a high degree of freedom in design of the flow path structure (Japanese Patent Application No. 2019-123741). Specifically, proposed is a fluid cleaning structure using a photocatalyst, which includes: a photocatalyst filter formed of a corrugated member having a plurality of crest parts and a plurality of trough parts alternately formed therein, the photocatalyst filter having a fluid passage hole for passing a fluid which is formed in one or both of a top potion of each crest part and a bottom portion of each trough part, the photocatalyst filter having a photocatalyst carried on obverse and reverse surfaces thereof; a light application part provided to one or both of one end side and the other end side in the direction along which the crest part and the trough part of the photocatalyst filter extend, the light application part being for applying ultraviolet light or visible light inwardly to the direction along which the crest part and the trough part extend; a fluid supply path provided on the side of one surface out of the obverse and reverse surfaces of the photocatalyst filter, the fluid supply path being for supplying a fluid toward the one surface; a fluid discharge path provided on the side of the other surface out of the obverse and reverse surfaces of the photocatalyst filter, the fluid discharge path being for discharging the fluid having been supplied from the fluid supply path to pass through the fluid passage hole and having exited from the other surface; and a dust collection filter provided to the interior of one or both of the fluid supply path and the fluid discharge path, and provided so as to face the one surface or the other surface.

In such a fluid cleaning structure, the light application part is not provided at the position opposed to the filter surface as in conventional art. Instead, the light application part is provided to one or both of one end side and the other end side in the direction along which each crest part and each trough part of the photocatalyst filter extend, and is configured to apply, from the position, ultraviolet light or visible light inwardly to the direction along which the crest part and the trough part extend. Therefore, the fluid can be caused to circulate in a straight manner in the filter thickness direction without being hindered by the light application part, the degree of freedom in design of a flow path is improved, and the unit including the filter and the light application part can be made significantly thin and compact.

However, in the above structure, light is applied inwardly to the direction from an end portion in the direction along which the crest part and the trough part of the photocatalyst filter extend. Therefore, when the area of the photocatalyst filter is made large, and the crest part and the trough part are elongated, light is less likely to sufficiently reach the depth in the inward direction. In particular, at a crest part/trough part away from the position of the light application part, light becomes insufficient. Thus, the light amount of the light application part needs to be increased, and the number of light application parts needs to be increased, whereby problems of cost increase and heat generation are posed.

In consideration of such a current situation, the present inventors conducted through studies and eventually found the following. That is, if the photocatalyst filter is not formed of a conventional fixed-type corrugated plate having crest parts and trough parts, but is formed such that an impeller in which a plurality of vane plates carrying a photocatalyst rotate upon receiving pressure from a fluid is provided, and light is applied from the outer circumferential side to the rotating vane plates of this impeller, it is possible to sequentially and uniformly apply light to the surface of each vane plate. Accordingly, it is possible to uniformly and efficiently supply light to the photocatalyst carrying portion by a small number of light application parts, and it is possible to obtain a sufficient cleaning effect as a whole, without increasing the light amount or increasing the number of light application parts. Then, the present inventors completed the present invention.

That is, the present invention encompasses the following features.
(1) A photocatalyst unit including: a photocatalyst filter including a fluid passage path through which a fluid passes, and a photocatalyst body carrier, in the fluid passage path, having a surface that comes into contact with the fluid and carries a photocatalyst; and a light application part that is provided inside the photocatalyst filter, and applies light composed of ultraviolet light or visible light toward the surface of the photocatalyst carrier, the surface carrying the photocatalyst, in which the photocatalyst carrier is an impeller having a plurality of vane plate parts each of which has a surface carrying the photocatalyst, the plurality of vane plate parts rotating about an axis upon receiving a pressure from the fluid passing through the fluid passage path, and the light application part is provided to apply the light to the rotating vane plate parts of the impeller, from a predetermined position on an outer circumferential side with respect to the vane plate parts of the impeller.
(2) The photocatalyst unit according to (1), in which the photocatalyst carrier is a propeller fan-like impeller that has: a shaft part that rotates about the axis; and a plurality of vane plate parts that are provided at an outer circumferential portion of the shaft part, each of the plurality of vane plate parts having a surface that carries the photocatalyst, and the fluid passage path has a fluid leading-in part that leads in the fluid from a direction along the axis, and a fluid leading-out part that leads out the fluid toward a downstream side in the direction along the axis.
(3) The photocatalyst unit according to (1) or (2), in which the photocatalyst filter has: a frame that is formed in a flat box shape having a small thickness relative to a size of each of upper and lower surfaces of the photocatalyst filter, and includes therein the fluid passage path; and a plurality of the impellers provided in parallel in a direction orthogonal to a thickness direction of the frame, and one or a plurality of the light application parts are provided for each impeller.
(4) The photocatalyst unit according to (3), in which the frame is a lattice-shaped frame having an upper surface and a lower surface, each of the upper surface and the lower surface having a gap through which a fluid passes, the impeller is provided so as to stand in a rotatable manner at a predetermined cross part provided in the lattice-shaped frame, and the light application part that applies light to the vane plate parts of the impeller is attached to another cross part in a surrounding of the cross part.
(5) The photocatalyst unit according to (4), in which a standing piece is provided to the other cross part, and the light application part is fixed to the standing piece.
(6) The photocatalyst unit according to any one of (3) to (5), in which the frame is provided with a rolling bearing that supports the impeller in a rotatable manner.
(7) The photocatalyst unit according to (1) or (2), in which the photocatalyst filter has: a cylindrical frame extending in a direction along the axis; and a plurality of the impellers arrayed on a shaft part extending in a length direction of the cylindrical frame, and one or a plurality of the light application parts are provided for each impeller.
(8) The photocatalyst unit according to (1), in which the photocatalyst carrier is a blower fan-like impeller that has: a shaft part that rotates about the axis; and a plurality of vane plate parts that are provided to an outer circumferential side of the shaft part, each of the plurality of vane plate parts having a surface that carries the photocatalyst, and the fluid passage path has a fluid leading-in part that leads in the fluid from a direction along the axis, or from one side in a direction orthogonal to the axis, and a fluid leading-out part that leads out the fluid in the direction along the axis, or toward another side in the direction orthogonal to the axis.
(9) The photocatalyst unit according to (1), in which the photocatalyst carrier is a cross flow fan-like impeller that has: a shaft part that rotates about the axis; and a plurality of vane plate parts that are provided on an outer circumferential side of the shaft part, each of the plurality of vane plate parts having a surface that carries the photocatalyst, and the fluid passage path has a fluid leading-in part that leads in the fluid from a direction orthogonal to the axis, and a fluid leading-out part that leads out the fluid from a position in the direction orthogonal to the axis, the position being is different from a position of the fluid leading-in part.
(10) The photocatalyst unit according to any one of (1) to (9), in which the light application part applies the light toward the axis of the impeller from the predetermined position.
(11) The photocatalyst unit according to any one of (1) to (10), including a reflection member that is provided on an outer circumferential side of the impeller, extends in a circumferential direction of the impeller, and has an inner circumferential surface that faces the impeller and serves as a reflection surface which reflects light.
(12) The photocatalyst unit according to any one of (1) to (11), further including a generator that generates power in accordance with rotation of the impeller, in which the light application part applies the light, using the power generated by the generator.
(13) A mask including the photocatalyst unit according to any one of (1), (2), or (8), in which the photocatalyst unit is provided in an air passage part that is generated in accordance with breathing of a mask wearer.
(14) The photocatalyst unit according to according to (11), in which the reflection member has an inner circumferential surface that has a substantially C-shape, in a cross-sectional view, which is concave in a curved manner on an inner circumferential side so as to cover the tip of each vane plate part of the impeller.
(15) The photocatalyst unit according to (11) or (14), in which the light application part is attached to the inner circumferential surface of the reflection member so as to penetrate the reflection member.
(16) The photocatalyst unit according to any one of (11), (14), or (15), in which the reflection member is provided in an annular shape so as to cover the impeller over a substantially entire circumference thereof.

### ADVANTAGEOUS EFFECTS OF THE INVENTION

According to the invention of the present application having the above configuration, the light application part provided at a position on the outer circumferential side applies light composed of ultraviolet light or visible light to the plurality of vane plate parts carrying the photocatalyst. Accordingly, hazardous substances, bad smell, or the like in the fluid can be efficiently decomposed/removed by the photocatalyst carried by the obverse and reverse surfaces of the filter and the inner surface of the fluid passage path. In addition, the light application part is not provided at a position opposed to the filter surface as in conventional art, but is provided at a position on the outer circumferential side of the vane plate parts as described above. Therefore, the fluid can circulate without being hindered by the light application part. Thus, the degree of freedom in design of a flow path is improved, and the unit including the filter and the light application part can be made significantly thin and compact.

Further, according to the present invention, it is possible to sequentially and uniformly apply light from the light application part to the surface of each vane plate part rotating under a fluid pressure. Accordingly, it is possible to uniformly and efficiently supply light to the photocatalyst carrying portion by a small number of light application parts, and it is possible to obtain a sufficient cleaning effect as a whole, without increasing the light amount or increasing the number of light application parts. Thus, cost increase can be avoided, the heat generation amount due to the light application parts can be suppressed, and the problem of heat can also be solved. In addition, since this impeller is not driven by a motor, the shaft supporting structure can be simplified, and, for example, can easily be configured to be removable. If the impeller can be removed, dirt such as oil attached to the wall surface or the like of the fluid passage path and the vane plate parts can be easily cleaned, or other maintenance work is facilitated.

Here, it is efficient that the light application part applies light toward the shaft part of the impeller from a predetermined position on the outer circumferential side, when applying light to the surfaces on both of upper and lower sides of each vane plate part.

In the photocatalyst unit, the photocatalyst carrier may be a propeller fan-like impeller that has: a shaft part that rotates about the axis; and a plurality of vane plate parts that are provided to an outer circumferential portion of the shaft part, each of the plurality of vane plate parts having a surface carrying the photocatalyst, and the fluid passage path may have a fluid leading-in part that leads in the fluid from a direction along the axis, and a fluid leading-out part that leads out the fluid toward a downstream side in the direction along the axis. In this case, when the impeller is installed in a fluid passage path in which a fluid is forcedly circulated by an electric fan or the like, or in an environment in which a fluid naturally circulates, the impeller can be smoothly rotated.

In the photocatalyst unit, the photocatalyst filter may have: a frame that is formed in a flat box shape having a small thickness relative to a size of each of upper and lower surfaces of the photocatalyst filter, and includes therein the fluid passage path; and a plurality of the impellers provided in parallel in a direction orthogonal to a thickness direction of the frame, and one or a plurality of the light application parts may be provided for each impeller. In this case, the number of impellers each being a photocatalyst carrier can be increased while the entirety is maintained to be thin, and the fluid cleaning effect by the photocatalyst can be enhanced. In addition, the number of impellers may be increased and the size per impeller may be reduced. In such a case, even when the passage amount (flow rate) of the fluid is small and the fluid pressure is small, the vane plate parts of each impeller can be caused to rotate, and light can be uniformly applied to the vane plate parts from a minimum number of light application parts, whereby the efficiency of fluid cleaning by the photocatalyst can be enhanced.

In the photocatalyst unit, the frame may be a lattice-shaped frame having an upper surface and a lower surface, each of the upper surface and the lower surface has a gap through which a fluid passes, the impeller may be provided so as to stand in a rotatable manner at a predetermined cross part provided in the lattice-shaped frame, and the light application part that applies light to the vane plate parts of the impeller may be attached to another cross part in a surrounding of the cross part. In this case, while the shaft part of the impeller and the light application part are provided to the cross parts in which rigidity is maintained, the fluid passing between the rotating vane plate parts can be efficiently caused to flow in or be discharged through the gap in the lattice-shaped frame, without being hindered by the light application part and the like. Accordingly, the pressure loss due to passage resistance of the fluid can be suppressed to be low, and further the weight of the unit can be reduced while the strength of the entirety of the unit is maintained.

In the photocatalyst unit, the frame may be provided with a rolling bearing that supports the impeller in a rotatable manner. In this case, even when dust and the like are accumulated on the shaft part, smooth rotation of the vane plate parts is maintained for a long period, and the pressure loss of the fluid can be reduced. In addition, even when the passage amount (flow rate) of the fluid is small and the fluid pressure is small, the vane plate parts of each impeller can be caused to rotate, and light can be uniformly applied to the vane plate parts from a minimum number of light application parts, whereby the efficiency of fluid cleaning by the photocatalyst can be enhanced.

In the photocatalyst unit, the photocatalyst filter may have: a cylindrical frame extending in a direction along the axis; and a plurality of the impellers arrayed on a shaft part extending in a length direction of the cylindrical frame, and one or a plurality of the light application parts may be provided for each impeller. In this case, it is possible to increase the chances of contact between the fluid and the photocatalyst carrier, without increasing the installation area of the photocatalyst unit in a direction orthogonal to the axis. Thus, a sufficient cleaning effect can be obtained.

In the photocatalyst unit, the photocatalyst carrier may be a blower fan-like impeller that has: a shaft part that rotates about the axis; and a plurality of vane plate parts that are provided to an outer circumferential side of the shaft part, each of the plurality of vane plate parts having a surface that carries the photocatalyst, and the fluid passage path may have a fluid leading-in part that leads in the fluid from a direction along the axis, or from one side in a direction orthogonal to the axis, and a fluid leading-out part that leads out the fluid in the direction along the axis, or toward another side in the direction orthogonal to the axis. In this case, in a state where light is being applied from the light application part toward the rotating vane plate parts, the fluid passing through the fluid passage path is brought into contact with the photocatalyst carried by the surfaces of the vane plate parts. Accordingly, similarly, hazardous substances, bad smell, or the like in the fluid can be efficiently decomposed/removed by the catalytic action of the photocatalyst. In addition, since the flowing direction of the fluid passing through the fluid passage path changes to the above-mentioned orthogonal direction, and the fluid temporarily stays between the rotating vane plate parts, the chances of contact between the fluid and the surfaces of the vane plate parts carrying the photocatalyst are increased, whereby the above-mentioned catalytic action can be enhanced. Since the fluid leading-out part is provided in a direction orthogonal to the fluid leading-in part as described above, variation in design can be increased.

In the photocatalyst unit, the photocatalyst carrier may be a cross flow fan-like impeller that has: a shaft part that rotates about the axis; and a plurality of vane plate parts that are provided on an outer circumferential side of the shaft part, each of the plurality of vane parts having a surface that carries the photocatalyst, and the fluid passage path may have a fluid leading-in part that leads in the fluid from a direction orthogonal to the axis, and a fluid leading-out part that leads out the fluid from a position in the direction orthogonal to the axis, which is different from a position of the fluid leading-in part. In this case, similarly, hazardous substances, bad smell, or the like in the fluid can be efficiently decomposed/removed by the catalytic action of the photocatalyst. In addition, since the time in which the fluid temporarily stays between the rotating vane plate parts can be made longer than that of the blower fan-like impeller, the chances of contact between the fluid and the surfaces of the vane plate parts carrying the photocatalyst are more increased. Thus, the above-mentioned catalytic action can be enhanced. Since the dimension in the axis direction of the vane plate part can be freely set, the fluid passage path can be made large in the axis direction. Thus, the impeller can be effectively rotated even when the wind pressure of the fluid passing through the fluid passage path is weak. In addition, the impeller can be efficiently installed in a relatively large flow path. Thus, it is also easy to suppress the circulation resistance to be lower.

The photocatalyst unit may include a reflection member that is provided, on an outer circumferential side of the impeller, extends in a circumferential direction of the impeller, and has an inner circumferential surface that faces the impeller and serves as a reflection surface which reflects light. In this case, light from the light application part can be efficiently applied as a whole to the surfaces of the vane plate parts of the impeller.

The photocatalyst unit may further include a generator that generates power in accordance with rotation of the impeller, and the light application part may apply the light, using the power generated by the generator. In this case, power can be supplied to the application part directly from the generator, or from a built-in battery which stores power generated by the generator, and thus the application part can apply light. Accordingly, there is no need to supply power from an external power supply to the light application part, and in addition, there is no need to provide rotation power for the impeller. Thus, no wiring or the like from an external power supply is required to be arranged. Accordingly, workability and the degree of freedom of installation at the time of post-installation of the photocatalyst unit into an existing duct or the like are significantly improved, whereby the usability can be significantly enhanced.

A mask of the present invention includes the above photocatalyst unit, and the photocatalyst unit is provided in an air passage part that is generated in accordance with breathing of a mask wearer. According to this mask, the impeller of the photocatalyst unit can be rotated in accordance with an air flow that occurs in accordance with breathing of the mask wearer. Then, in a state where light is being applied to the vane plate parts of the impeller from the light application part, air is brought into contact with the photocatalyst carried by the surfaces of the vane plate parts. Accordingly, hazardous components, bad smell components, or the like in the air can be efficiently decomposed/removed by the catalytic action of the photocatalyst, and viruses or the like in the air can be inactivated. Then, air is led out from the air leading-out part on the mask rear side or from the air leading-out part on the mask front side. Thus, there is an advantage that viruses or the like can be effectively prevented from entering the human body or being diffused into the atmosphere.

In the photocatalyst unit, the reflection member may have an inner circumferential surface that has a substantially C-shape, in a cross-sectional view, which is concave in a curved manner on an inner circumferential side so as to cover the tip of each vane plate part of the impeller. In this case, light from the light application part can be more efficiently applied as a whole to the surfaces of the vane plate parts of the impeller, and leakage of light from the upper and lower fluid entrance and exit of the unit can be prevented as well.

In the photocatalyst unit, the light application part may be attached to the inner circumferential surface of the reflection member so as to penetrate the reflection member. In this case, light from the light application part can be more assuredly applied toward the vane plate parts. In addition, heat generated by the light application part can be transmitted to the reflection member, to be dissipated into the fluid passing along the inner surface of the reflection member.

In the photocatalyst unit, the reflection member may be provided in an annular shape so as to cover the impeller over a substantially entire circumference thereof. In this case, light from the light application part can be more efficiently applied as a whole to the surfaces of the vane plate parts of the impeller.

### BRIEF DESCRIPTION OF THE DRAWINGS

[FIG. 1] FIG. 1 is a perspective view showing a photocatalyst unit according to a first embodiment of the present invention.
[FIG. 2] FIG. 2 is a perspective view showing a state where an upper lid of the photocatalyst unit is removed.
[FIG. 3] FIG. 3 is a perspective view showing a state where reflection members are further removed.
[FIG. 4] FIG. 4 is a plan view of the state shown in FIG. 3, in which the upper lid and the reflection members are indicated by imaginary lines.
[FIG. 5A] FIG. 5A is an A-A cross-sectional view of FIG. 4.
[FIG. 5B] FIG. 5B is a B-B cross-sectional view of FIG. 4.
[FIG. 6] FIG. 6 is a bottom view of the photocatalyst unit
[FIG. 7] FIG. 7 is a perspective view showing a main part of the photocatalyst unit.
[FIG. 8] FIG. 8 is an explanatory view showing a use state of the photocatalyst unit.
[FIG. 9] FIG. 9 is a partial cross-sectional view showing a photocatalyst unit according to another embodiment of the present invention.
[FIG. 10] FIG. 10 is a perspective view showing a photocatalyst unit according to a second embodiment of the present invention.
[FIG. 11] FIG. 11 is a plan view of the photocatalyst unit.
[FIG. 12] FIG. 12 is a cross-sectional view of the photocatalyst unit.
[FIG. 13] FIG. 13 is a perspective view showing an air cleaner provided with the photocatalyst unit of the second embodiment.
[FIG. 14] FIG. 14 is a cross-sectional view showing a photocatalyst unit according to a third embodiment of the present invention.
[FIG. 15] FIG. 15 is a cross-sectional view showing a photocatalyst unit according to a fourth embodiment of the present invention.
[FIG. 16] FIG. 16 is a cross-sectional view showing a modification of the photocatalyst unit according to the fourth embodiment.
[FIG. 17] FIG. 17 is a perspective view showing a photocatalyst unit according to a fifth embodiment of the present invention.
[FIG. 18] FIG. 18 is a plan cross-sectional view of the photocatalyst unit.
[FIG. 19] FIG. 19 is a side cross-sectional view of the photocatalyst unit
[FIG. 20] FIG. 20 is a perspective view showing an example in which the flow of a fluid is reversed in the photocatalyst unit shown in FIG. 14.
[FIG. 21] FIG. 21 is a perspective view of a photocatalyst unit according to a sixth embodiment of the present invention.
[FIG. 22] FIG. 22 is a plan cross-sectional view of the photocatalyst unit.
[FIG. 23] FIG. 23 is a side cross-sectional view of the photocatalyst unit.
[FIG. 24] FIG. 24 is a perspective view showing an embodiment of a mask according to the present invention.
[FIG. 25] FIG. 25 is a perspective view showing another embodiment of the mask according to the present invention.

### DESCRIPTION OF EMBODIMENTS

Next, a first embodiment of a photocatalyst unit according to the present invention is described in detail with reference to the attached drawings.

### (First embodiment)

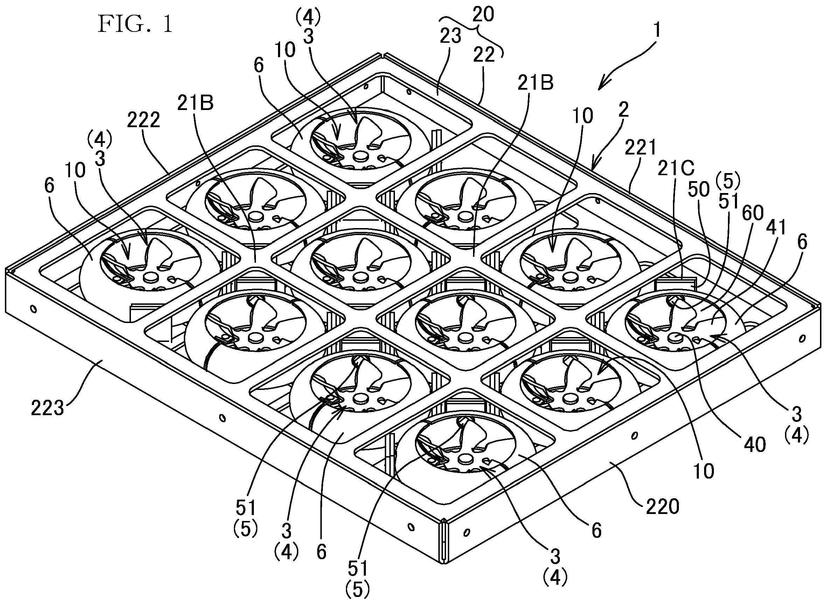

As shown in FIG. 1 to FIG. 7, a photocatalyst unit 1 according to the first embodiment of the present invention includes: a photocatalyst filter 2 which includes a lattice-shaped frame 20 having a flat box body shape in which the thickness dimension is smaller than the lengthwise and widthwise dimensions of the upper and lower surfaces thereof, and photocatalyst carriers 3 each of which has a surface that come into contact with a fluid and carries a photocatalyst; and light application parts 5 which are provided inside the photocatalyst filter 2 and which each apply light composed of ultraviolet light or visible light toward the surface of a corresponding photocatalyst carrier 3. The photocatalyst unit 1 can efficiently decompose/remove hazardous substances, bad smell, or the like in a fluid by the photocatalyst on the photocatalyst carrier.

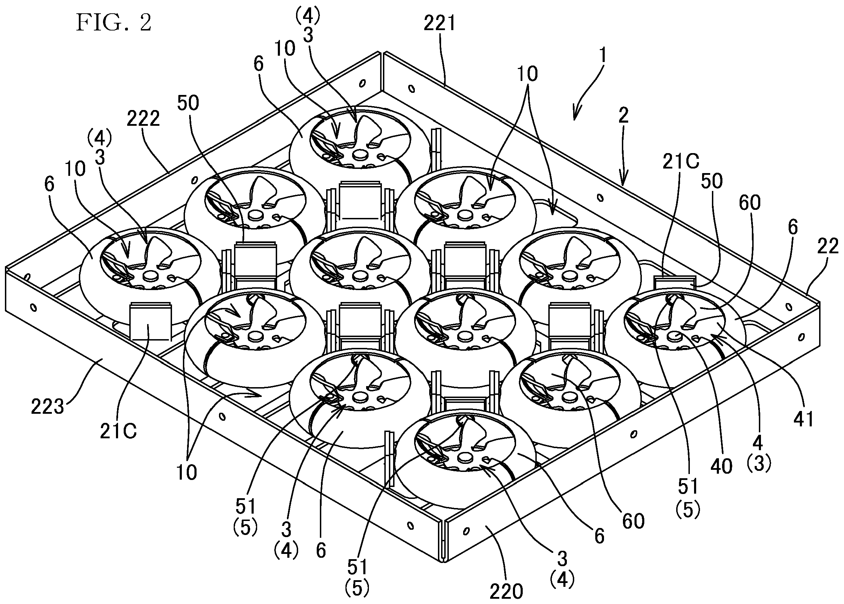

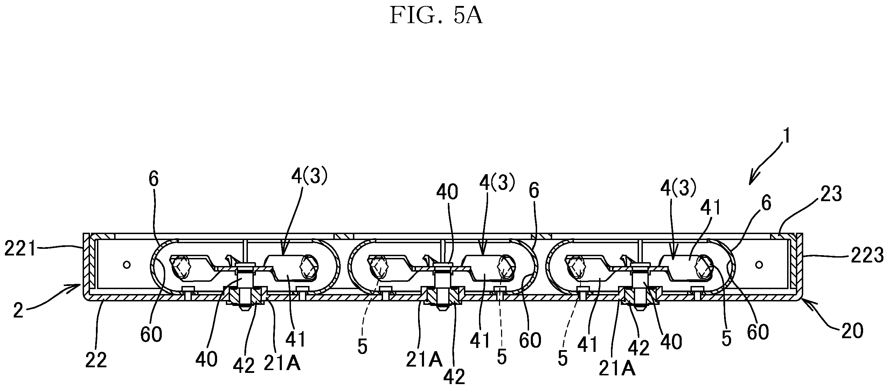

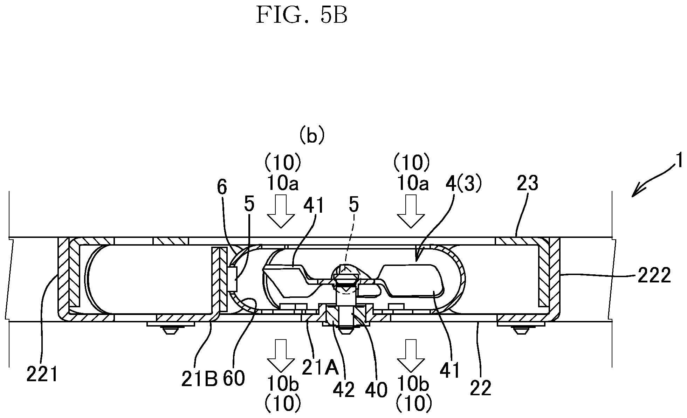

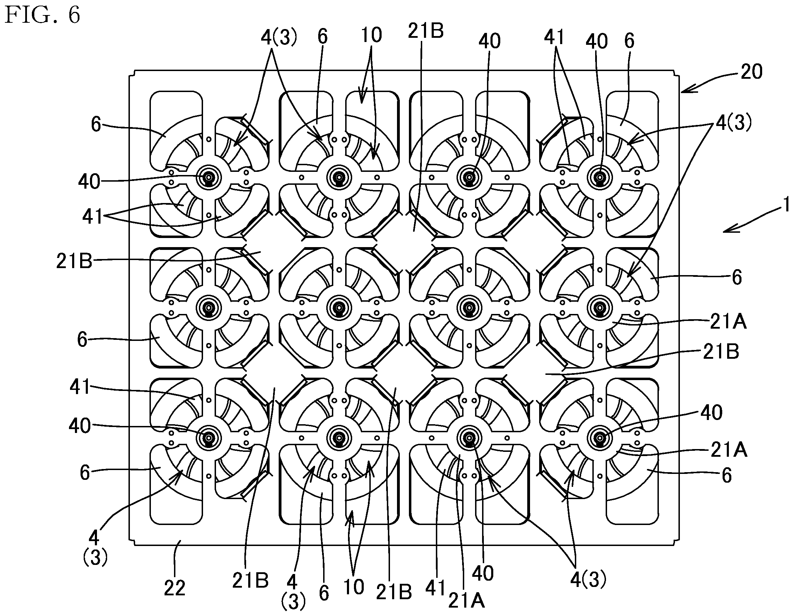

The lattice-shaped frame 20 has a frame body 22 (see FIG. 2 and FIG. 6) forming the lower surface thereof, and an upper lid 23 (see FIG. 1 and FIGS. 5A, 5B) forming the upper surface. The lattice-shaped frame 20 has therein fluid passage paths 10 in the thickness direction so that a fluid such as air can pass, in the thickness direction, the inside of each of the fluid passage paths through the lattice-shaped gap between the upper and lower surfaces. The frame body 22 and the upper lid 23 forming the lattice-shaped frame 20 are each made of metal. The upper lid 23 is fitted in a state of being in contact with the inner surface of side walls 220, 221, 222, 223 of the lattice-shaped frame 20, thereby being integrally assembled. Accordingly, heat generated by each light application part 5 is absorbed by the entire frame, and then efficiently dissipated into the fluid. As shown in FIG. 5B, the fluid passage path 10 has: a fluid leading-in part 10a which leads in a fluid from a direction along the axis of an impeller 4 described later, from the upper surface side of the lattice-shaped frame 20; and a fluid leading-out part 10b which leads out the fluid toward the downstream side in the direction along the axis.

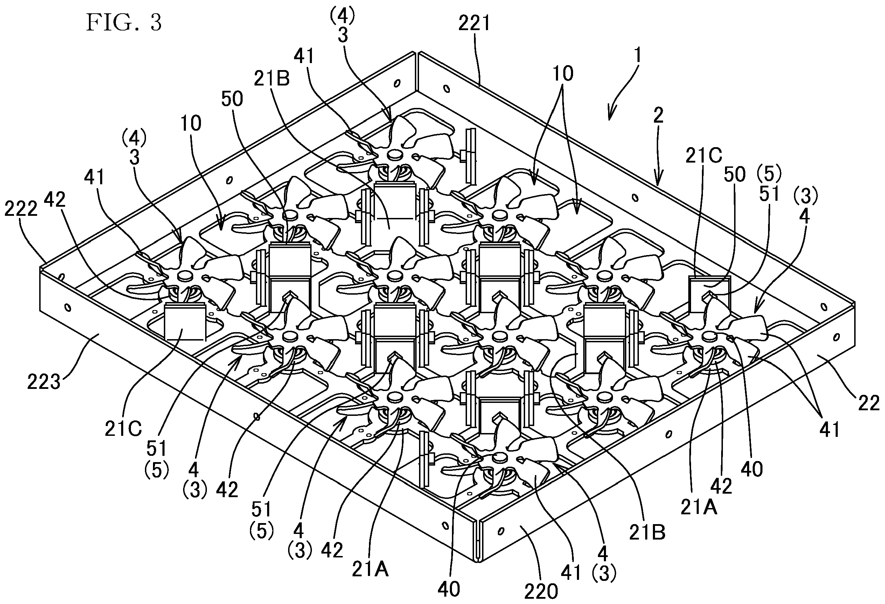

As seen from FIG. 3, FIG. 4, and FIG. 6, each photocatalyst carrier 3 is mounted to a predetermined cross part 21A, which is rigid, of the lattice-shaped frame 20 on the lower surface side, and the light application part 5 is mounted to a cross part 21B, which is also rigid, in the vicinity thereof. Therefore, without being hindered by the light application part 5, etc., a fluid can efficiently flow in or be discharged through the gap in the lattice-shaped frame 20, the pressure loss due to passage resistance of the fluid can be suppressed to be low, and further the unit has a reduced weight while a sufficient strength is maintained.

The photocatalyst carrier 3 is a propeller fan-like impeller 4 that includes: a shaft part 40 which rotates about the axis extending in the thickness direction of the lattice-shaped frame 20; and a plurality of vane plate parts 41 which are provided in an outer circumferential portion of the shaft part 40 and respectively have the surfaces carrying the photocatalyst. In the impeller 4, these plurality of vane plate parts 41 rotate upon receiving a pressure from the fluid passing through the fluid passage path 10. That is, the impeller 4 is configured so that the plurality of vane plate parts 41 rotate like a wind mill upon receiving a pressure of the fluid that is led in from the fluid leading-in part 10a, and led out the fluid to the fluid leading-out part 10b.

More specifically, the shaft part 40 of the impeller 4 is provided so as to stand in a rotatable manner at a predetermined cross part 21A of the lattice-shaped frame 20 forming the upper surface or the lower surface, and the light application part 5 which applies light to the vane plate parts 41 of the impeller 4 is attached to another cross part 21B that is in a surrounding of the cross part 21A. In the present embodiment, the shaft part 40 of the impeller 4 is supported in a rotatable manner by a rolling bearing 42 fixed to the frame body 22 forming the lower surface of the lattice-shaped frame 20, whereby the vane plate parts 41 can be smoothly rotated. It is noted that the rolling bearing 42 may be provided to the upper lid 23 of the lattice-shaped frame 20, or alternatively, the rolling bearing 42 may be provided to both of the frame body 22 and the upper lid 23.

In the present example, the shaft part 40 and the vane plate parts 41 are each made of metal. In particular, a plurality of vane plate parts 41 are integrally formed by subjecting a metal plate to press working and then having the upper and lower surfaces thereof coated by a photocatalyst layer. That is, the plurality of vane plate parts 41 are formed as a single-piece vane member in which the plurality of vane plate parts 41 are formed with a predetermined interval therebetween so as to extend in the radial direction from the center portion thereof having a mounting hole therein. This mounting hole is clamped to an outer circumferential portion of a tip portion of the shaft part 40, whereby the shaft part 40 and the vane member having the vane plate parts 41 are integrated with each other.

Since the plurality of vane plate parts 41 are formed as such a single-piece integrated processed article, the strength of the entirety of the vane member is maintained, and at the same time, reduction of the thickness and weight thereof is enabled. As the metal material of the shaft part 40 and the vane plate parts 41, various metal raw materials such as aluminum, stainless steel, etc., can be used, but are not limited thereto. A raw material other than metal may be used.

The photocatalyst layer is a layer obtained by causing the surface of a member to carry photocatalyst particles such as ultraviolet light excitation-type photocatalyst particles such as titanium oxide, or visible light excitation-type photocatalyst particles of which the main component is tungsten trioxide. The method of carrying (formation of photocatalyst layer) the photocatalyst particles is not limited in particular, but a slurry immersion impregnation method which costs relatively less is preferable. Means such as another immersion impregnation method, a vacuum impregnation method, or a sol-gel method can also be used.

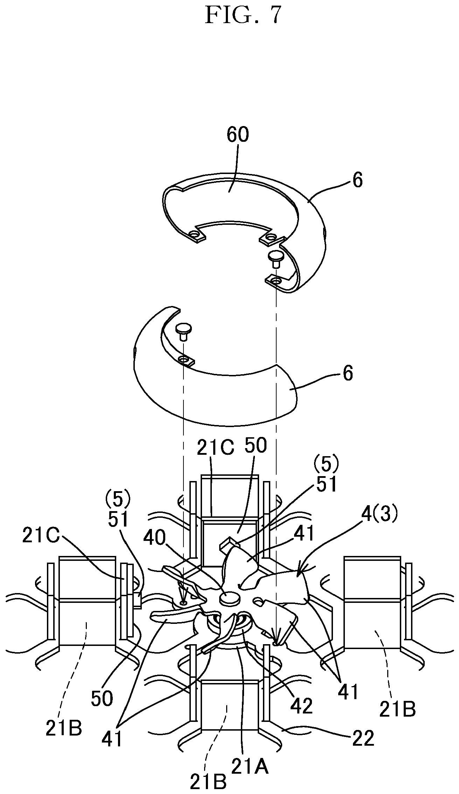

The light application part 5 is provided so as to apply the light to the rotating vane plate parts 41 of the impeller 4, from a predetermined position on the outer circumferential side with respect to the vane plate parts 41 of the impeller 4. Specifically, as shown in FIG. 7, the cross part 21B is provided with a standing piece 21C, and the light application part 5 is fixed to this standing piece 21C. Therefore, heat of the light application part 5 is transmitted to the entirety of the lattice-shaped frame 20 through the standing piece 21C, and is efficiently dissipated in the fluid passing through the gap in the frame.

For the light application part 5, an LED substrate 50 having an LED element 51 as a light source that applies light by use of power supplied from a power supply, etc., (not shown), is used for example. However, the present invention is not limited thereto. In the present example, two light application parts 5 are respectively provided at angular positions shifted by 90 degrees from each other. Due to rotation of the plurality of vane plate parts 41 of the impeller 4, the plurality of vane plate parts 41 sequentially receive light, whereby light is uniformly applied to the entirety of the surfaces of all the vane plate parts 41.

That is, in the case of a conventional photocatalyst filter, it is necessary to provide an application part which applies light to the surface of a photocatalyst carrier in a number that allows simultaneous light application to the entire surface in accordance with the size of the surface. However, in the present invention, since the photocatalyst carrier 3 implemented as the impeller 4 rotates, light is sequentially applied from the light application part 5 to the surface of the vane plate parts 41 at the time point when the vane plate parts 41 and the shaft part 40 have come close to the installation portion of the light application part 5. Therefore, it is possible to uniformly apply light to the entirety of the surfaces of all the vane plate parts 41 by merely providing about one or two light application parts 5.

The light application direction of the light application part 5 is set so as to apply light toward the shaft part 40 of the impeller 4 from the predetermined position. However, light may be applied in a diagonal direction shifted with respect to the shaft part 40. Light may be applied from diagonally above or diagonally below. However, in order to uniformly apply light to the entirety of the surfaces of all the vane plate parts 41 as described above, that is, to both of the upper surfaces of the vane plate parts 41 positioned on the upstream side of the passage direction of the fluid, and the lower surfaces of the vane plate parts 41 positioned on the downstream side of the passage direction of the fluid, it is preferable to apply light in a direction at a right angle with respect to the axis of the shaft part 40.

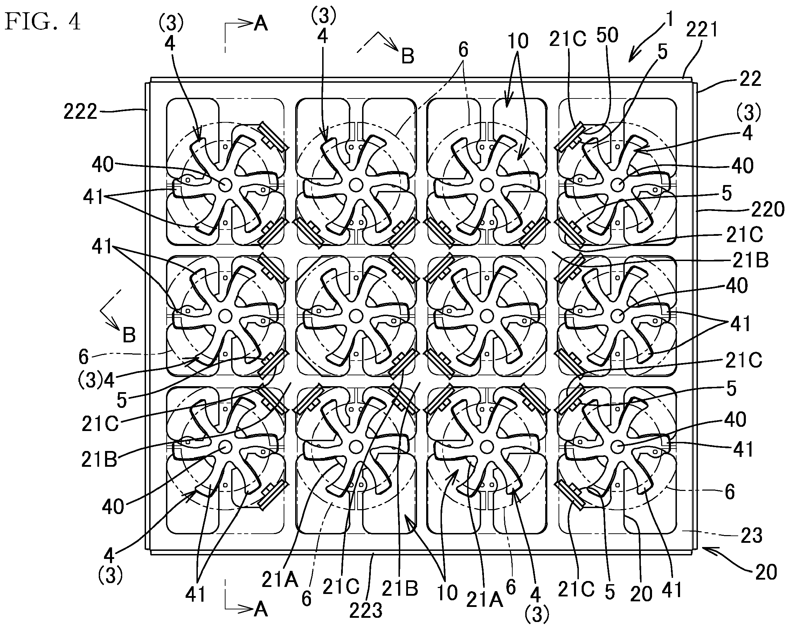

In the present example, a plurality of impellers 4 are provided in parallel in directions orthogonal to the filter thickness direction, and one, two, or more light application parts 5 are provided for each impeller 4. Specifically, a total of 12 impellers 4 are provided, i.e., 3 × 4 in the lengthwise direction and the widthwise direction of the lattice-shaped frame 20. The shaft parts 40 of the impellers 4 are provided at substantially equal intervals in the lengthwise/widthwise directions at the cross parts 21A of the lattice-shaped frame 20. A light application part 5 is provided at a cross part 21B diagonally adjacent to the cross part 21A where each shaft part 40 is provided. In the present example, as also seen from FIG. 4, two or more light application parts 5 corresponding to two or more different impellers 4 are provided to one cross part 21B, whereby efficient configuration is realized.

The number and array of the impellers 4 are not limited to the form according to the present example. For example, as shown in a second embodiment described later, only one impeller 4 may be provided to the photocatalyst filter. When two or more impellers 4 are provided as well, the impellers 4 may be disposed in one row or in another array, e.g., at random, other than the 3 × 4 array.

On the outer circumferential side of each impeller 4, provided is a metal-made reflection member 6 which extends in the circumferential direction of the impeller 4 and has the inner circumferential surface that faces the impeller 4 and serves as a reflection surface 60 which reflects light. Examples of the reflection surface 60 include the metal raw material surface of the inner circumferential surface of the reflection member 6 as is, a surface obtained by processing the metal raw material surface into a mirror surface, a surface obtained by attaching a mirror surface sheet to the inner circumferential surface, and a surface obtained by applying a reflective raw material to the inner circumferential surface is used.

As also shown in FIG. 5A and FIG. 5B, the reflection member 6 is shaped so as to have an inner circumferential surface (60) that has a substantially C-shape, in a cross-sectional view, which is concave in a curved manner on the inner circumferential side so as to cover the tip of each vane plate part 41 of the impeller 4. Accordingly, light from the light application part 5 can be more efficiently applied as a whole to the surfaces of the vane plate parts 41 of the impeller 4, and leakage of light from the upper and lower fluid entrance and exit of the unit can be prevented as well. As for the substantially C-shape, in the present example, the inner circumferential surface has an arc shape in a cross-sectional view. However, various shapes such as a polygonal shape or a combination of a plane and a curved surface, can be adopted.

The reflection member 6 is provided in an annular shape so as to cover the impeller 4 over a substantially entire circumference thereof. In the present example, as shown in FIG. 7, the reflection member 6 is configured by combining two division members that each cover a substantially semicircle. When the impeller 4 is covered over a substantially entire circumference thereof in this manner, light from the light application part 5 can be more efficiently applied as a whole to the surfaces of the vane plate parts 41 of the impeller 4. It is noted that the present invention is not limited thereto, and the reflection member 6 may be partially discontinued, or may be provided in an intermittent manner.

As shown in FIG. 5B, the light application part 5 is configured such that the light application part 5 is disposed in a state of being substantially in close contact with the outer circumferential surface side of the reflection member 6, and only a light-emitting element 51 thereof penetrates the reflection member 6 to the inner circumferential surface (60) side to apply light to the impeller 4. Instead of causing the light-emitting element 51 to penetrate, the reflection member 6 may be configured to be partially discontinued, and the light application part 5 may be provided at the discontinued position. Alternatively, the light application part may be provided to an inner-side position of the reflection member.

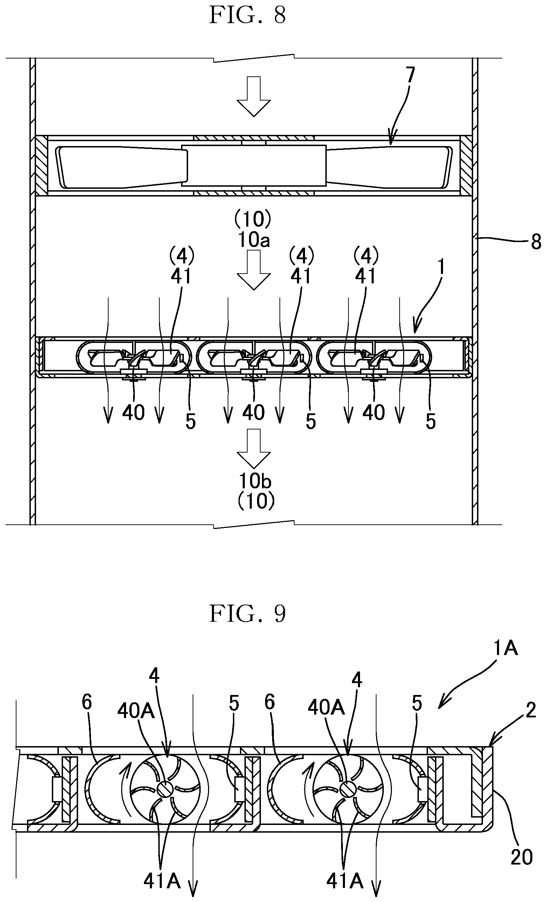

In the photocatalyst unit 1 according to the present invention, as described above, the impeller 4 which rotates upon receiving a pressure from a fluid is used as a photocatalyst carrier, to which light is applied from the light application part 5, whereby light is uniformly and efficiently applied to the entirety of the rotating vane plate parts 41, thereby efficiently obtaining a fluid cleaning action by a photocatalyst. As an example of usage form, as shown in FIG. 8, the photocatalyst unit 1 can function by being installed along a fluid passage path 10 in which a fluid is forcedly circulated by an electric fan 7 or the like in a ventilation duct 8 or the like that performs ventilation in a building. However, the usage form is by no means limited to providing the photocatalyst unit 1 in an environment in which a fluid is forcedly circulated, and it is understood that the photocatalyst unit 1 can function by being provided in an environment, for example, in which a fluid naturally circulates.

Although an embodiment of the present invention has been described above, the present invention is by no means limited to the embodiment. For example, in the example described above, an example (an example of an impeller of a so-called "horizontal axis type" in which the axis is set to be parallel to the direction along which a fluid flows) in which the impeller is composed of: a shaft part which rotates about the axis extending in the thickness direction; and a plurality of vane plate parts provided to an outer circumferential portion of the shaft part and carrying the photocatalyst on the surfaces thereof, has been described. However, as shown in FIG. 9, the impeller may be an example (an impeller of a so-called "perpendicular axis type" in which the axis is set in a direction orthogonal to the direction along which a fluid flows) composed of: a shaft part 40A which rotates about the axis extending in a direction orthogonal to the thickness direction; and a plurality of vane plate parts 41A which rotate about the shaft part 40A. It is understood that, other than these, the present invention can be implemented in various forms without departing from the gist of the present invention.

### (Second embodiment)

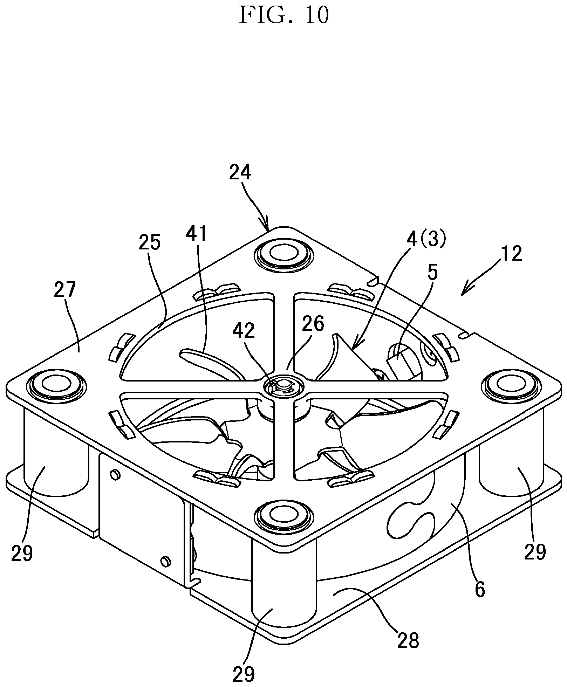

FIG. 10 to FIG. 12 show a photocatalyst unit 12 according to a second embodiment of the present invention. The photocatalyst unit 12 includes a support frame 24 formed in a flat box shape, and a single photocatalyst carrier 3 implemented as a propeller fan-like impeller 4 provided in this support frame 24. The support frame 24 includes: an upper surface plate 27 and a lower surface plate 28 which are each provided with an opening portion 25 for allowing a fluid to circulate therethrough and a lattice-shaped part 26 provided with a rolling bearing 42 which rotatably supports the impeller 4; and a coupling member 29 which couples the upper surface plate 27 and the lower surface plate 28 with each other.

The support frame 24 is provided with a fluid passage path 10 that includes: a fluid leading-in part 10a which leads in a fluid from the opening portion 25 of the upper surface plate 27 to the inside of the support frame 24; and a fluid leading-out part 10b which leads out the fluid from the opening portion 25 of the lower surface plate 28 to the outside of the support frame 24. In addition, the photocatalyst unit 12 is provided with: a light application part 5 which applies light composed of ultraviolet light or visible light toward the surface, specifically, the surfaces of the vane plate parts 41 and the shaft part 40 of the impeller 4, of the photocatalyst carrier 3 carrying a photocatalyst; and a reflection member 6 which extends in the circumferential direction of the impeller 4 and has the inner circumferential surface facing the impeller 4, as a reflection surface which reflects light.

Then, light of the light application part 5 is applied from a predetermined position on the outer circumferential side with respect to the vane plate parts 41 of the impeller 4, toward the rotating vane plate part 41 of the impeller 4, preferably toward the shaft part 40, in a direction at a right angle with respect to the axis of the shaft part 40. Under this state, the fluid passing through the fluid passage path 10 is brought into contact with the photocatalyst carried by the surfaces of the vane plate parts 41, and the like. Accordingly, hazardous substances, bad smell, or the like in the fluid can be efficiently decomposed/removed by the catalytic action of the photocatalyst.

The photocatalyst unit 12 according to the second embodiment can function by being provided, for example, in the inside of a ventilation duct in which a fluid is forcedly circulated, in an environment in which a fluid naturally circulates, or the like. In addition, when compared with the photocatalyst unit 1 according to the first embodiment in which a plurality of impellers 4 are arrayed in directions orthogonal to the thickness direction of the frame, the photocatalyst unit 12 of the second embodiment can be made more compact as a unit. Further, in accordance with the necessary fluid flow rate and flow path size, the form of the installation place, and the like, a plurality of the units can be freely combined in the lengthwise/widthwise directions, thereby being able to efficiently configure devices in various forms. Further, even in a case where the amount of the fluid passing through the fluid passage path 10 is small, the impeller 4 can be smoothly rotated.

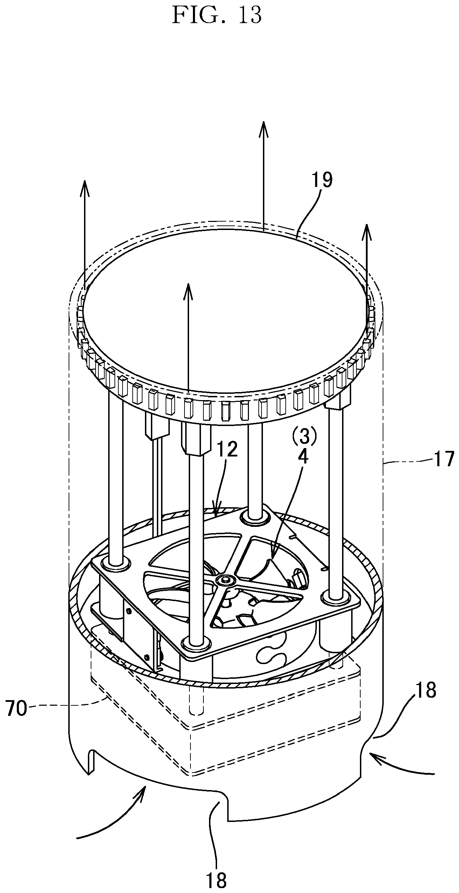

For example, FIG. 13 shows a desktop-type air cleaner in which: the photocatalyst unit 12 shown in FIG. 10 to FIG. 12 is provided in a cylindrical case 17 in a coaxial manner, with the shaft part 40 of the photocatalyst unit 12 being aligned with the center axis of the cylindrical case 17; and a small electric fan 70 is also coaxially installed below the photocatalyst unit 12. According to this air cleaner, a fluid led into the cylindrical case 17 from an opening 18 provided in a lower part of cylindrical case 17, in accordance with the suction force of the electric fan 70, is caused to pass through the installation portion of the photocatalyst unit 12, to be led out to the outside of cylindrical case 17 from an opening 19 provided in an upper part of the cylindrical case 17. Thus, with a significantly compact configuration, hazardous substances, bad smell, or the like in the fluid can be efficiently decomposed/removed by the catalytic action of the photocatalyst carried by the impeller 4. In the present example, the electric fan 70 which takes air into the case is provided in a lower end portion of the case, but may be provided in an upper end portion or at an intermediate position. Also, the configuration regarding the positions of leading in and leading out of air is by no means limited to a configuration in which air is led in from below and is exhausted from an upper part as in the present example. In the cylindrical case 17, four support column parts 171 which are inserted in insertion holes 240 provided at four corners of the photocatalyst unit 12, for fixing the position of the photocatalyst unit 12 are provided in parallel to the axis of the cylindrical case 17. In the present example, only one photocatalyst unit 12 is provided. However, in the present example, when these support column parts 171 are inserted into the insertion holes 240 in a similar manner, two or more photocatalyst units 12 can be provided in combination along the axis direction.

### (Third embodiment)

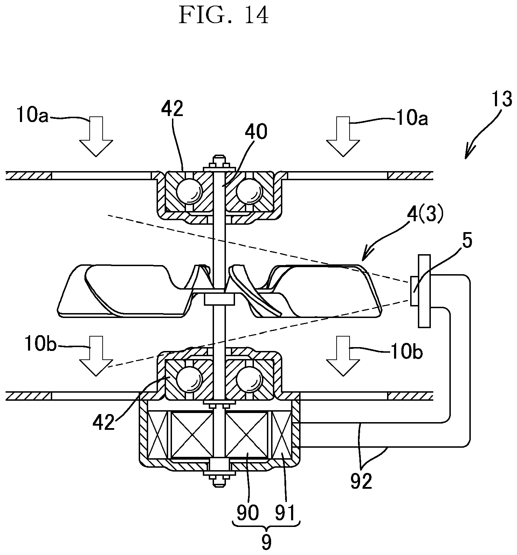

FIG. 14 shows a photocatalyst unit 13 according to a third embodiment of the present invention. This photocatalyst unit 13 is provided with a generator 9 which generates power in accordance with rotation of the impeller 4. The generator 9 includes: an armature core 90 which rotates integrally with the shaft part 40 of the impeller 4 forming the photocatalyst carrier 3; and a field winding 91 provided on the outer circumferential side of the armature core 90. Power generated by the generator 9 is supplied to the light application part 5 via a conductive wire 92, whereby light composed of ultraviolet light or visible light is applied from the light application part 5 toward the axis and the like of the impeller 4. According to this configuration, there is no need to supply power to the light application part 5 from a separate power supply or the like provided at a separated position. Thus, the photocatalyst unit 13 can be more simplified, the installation work is significantly facilitated, and a range of usage can be dramatically improved. In addition, in a system in which light is applied using power generated by the generator 9, when rotation of the shaft part 40 becomes fast and the flow rate of the passing fluid is increased, the power generated by the generator 9 is also increased, and light can be applied at a higher illuminance. Accordingly, a very efficient system is realized.

### (Fourth embodiment)

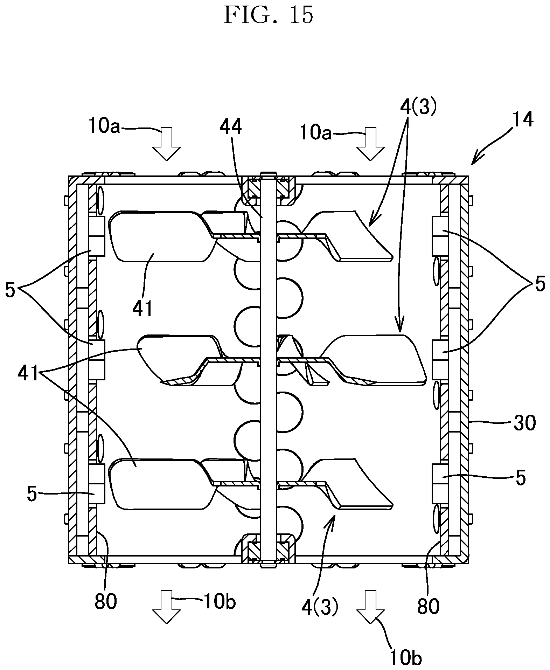

FIG. 15 shows a photocatalyst unit 14 according to a fourth embodiment of the present invention. This photocatalyst unit 14 includes: a cylindrical frame 30 extending in the direction along the axis of the impeller 4; and a plurality of (three in the example shown in FIG. 15) the impellers 4 arrayed on one shaft part 44 extending in the length direction of the cylindrical frame 30. For each impeller 4, one, two, or more light application parts 5 which each apply light, from a predetermined position on the outer circumferential side with respect to the vane plate parts 41 of the impeller 4, toward the rotating vane plate parts 41, preferably, toward the shaft part 44 in a direction at a right angle with respect to the axis thereof, are provided. In addition, in the cylindrical frame 30, a reflection member 80 extending in the length direction along the inner circumferential surface of the cylindrical frame 30 is provided. Further, on the shaft part 44 of the impeller 4, a generator (not shown) which generates power in accordance with rotation of the impeller 4 and supplies the power to the light application part 5 is provided as necessary.

According to this configuration, for example, when the amount of the fluid passing through the fluid passage path composed of the fluid leading-in part 10a and the fluid leading-out part 10b is large, or when the flow speed is fast, it is possible to increase the chances of contact between the fluid and the photocatalyst carrier 3 without increasing the installation area of the photocatalyst unit 14 in a direction orthogonal to the axis of the impeller 4, thereby being able to obtain a sufficient cleaning effect.

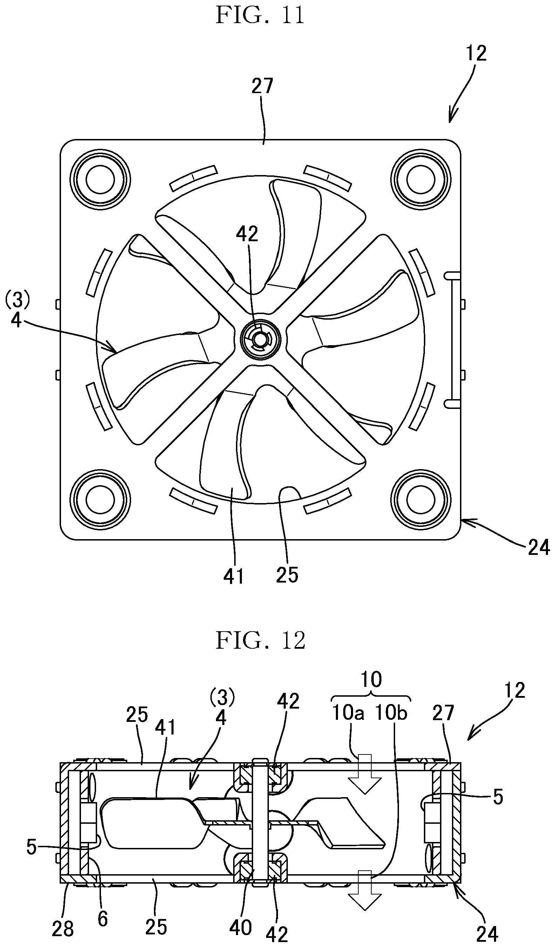

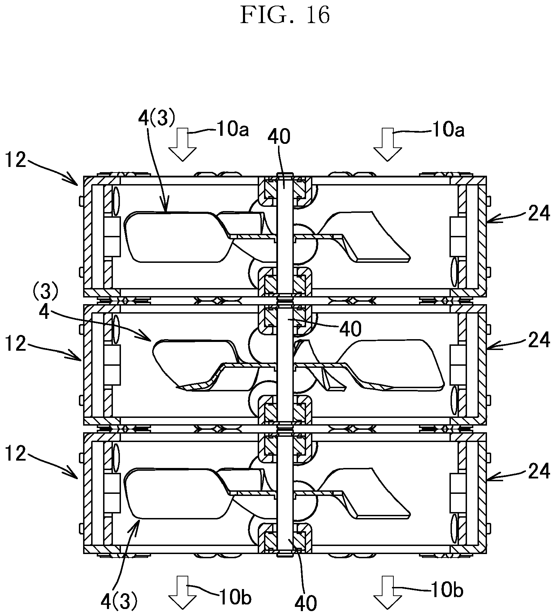

Although already described with reference to the example of the cylindrical case accommodation form shown in FIG. 13, instead of the above-described embodiment in which a plurality of impellers 4 are arrayed on one shaft part 44 extending in the length direction of the cylindrical frame 30 as shown in FIG. 15, a plurality of photocatalyst units 12 (see the second embodiment shown in FIG. 11) in each of which a photocatalyst carrier 3 implemented as a single impeller 4 is provided in the support frame 24 formed in a flat box shape, may be arrayed so as to be stacked in the axis direction of the impeller 4, as shown in FIG. 16. In this configuration as well, it is possible to increase the chances of contact between the fluid and the photocatalyst carrier 3 without increasing the installation area of the photocatalyst unit 12 in a direction orthogonal to the passage direction of the fluid. In the example in FIG. 15, a plurality of impellers 4 are fixed to one shaft part 44, and the plurality of impellers 4 are integrally rotated via the shaft part 44. However, a part or all of the plurality of impellers 4 may be independently supported so as to be rotatable with respect to the shaft part 44, to independently rotate.

### (Fifth embodiment)

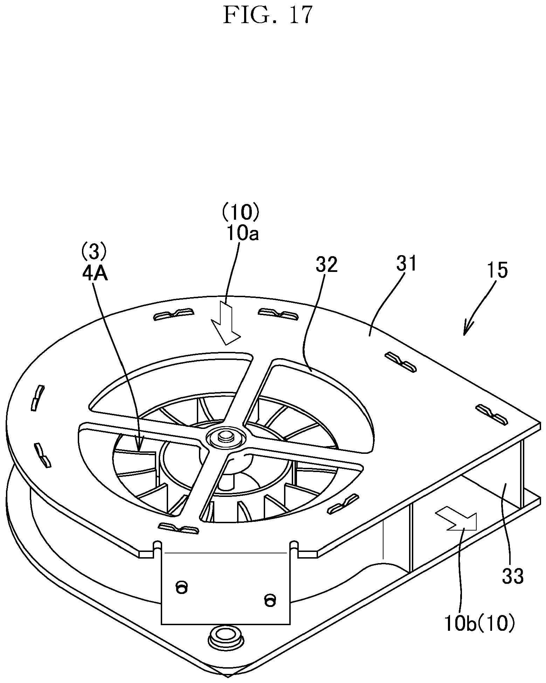

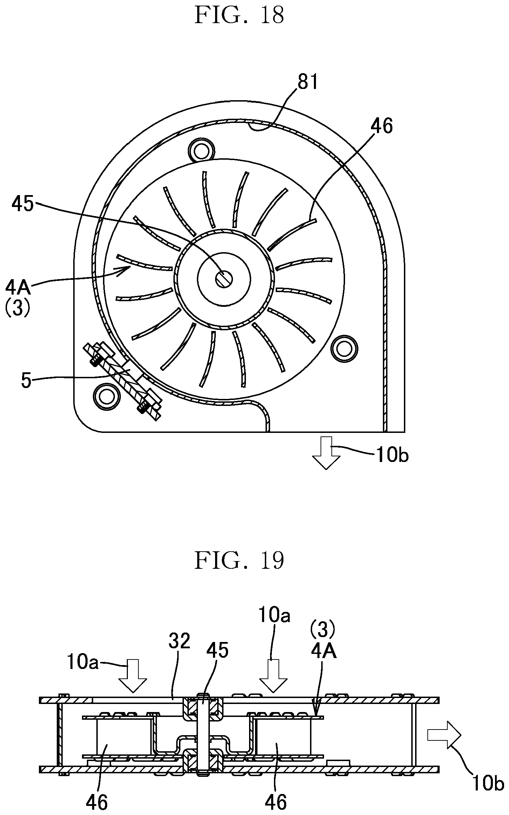

FIG. 17 to FIG. 19 show a photocatalyst unit 15 according to a fifth embodiment of the present invention. This photocatalyst unit 15 is configured such that a blower fan-like impeller 4A which includes: a shaft part 45 serving as the center of rotation of the photocatalyst carrier 3; and a plurality of vane plate parts 46 which are provided on the outer circumferential side of the shaft part 45 and has the surfaces carrying a photocatalyst, is provided in a flat box-shaped frame 31. In addition, the photocatalyst unit 15 is provided with: a light application part 5 which applies light, from a predetermined position on the outer circumferential side with respect to the vane plate parts 46 of the impeller 4A, toward the rotating vane plate part 41, preferably, toward the shaft part 45 in a direction at a right angle with respect to the axis thereof; and a reflection member 81 which extends in the circumferential direction of the impeller 4A and has the inner circumferential surface facing the impeller 4A, which is used as a reflection surface reflecting light.

The box-shaped frame 31 is provided with a fluid passage path 10 which includes: a fluid leading-in part 10a which leads in a fluid through an opening portion 32 formed in the upper surface of the box-shaped frame 31, from a direction along the axis of the impeller 4A; and a fluid leading-out part 10b which leads out the fluid through an opening portion 33 formed in a lateral surface of the box-shaped frame 31, toward a direction orthogonal to the axis. The impeller 4A has a shape substantially similar to those of the vanes of a blower fan (also referred to as a turbofan) that discharges a fluid by centrifugal force into a direction orthogonal to the rotation axis. The impeller 4A rotates upon receiving a pressure of a fluid led in through the fluid leading-in part 10a in the axis direction of the impeller 4A, thereby leading out the fluid through the fluid leading-out part 10b toward a direction orthogonal to the axis of the impeller 4A.

According to this configuration, in a state where light is being applied from the light application part 5 toward the rotating vane plate parts 41, the fluid passing through the fluid passage path 10 is brought into contact with the photocatalyst carried by the surfaces of the vane plate parts 46 and the like. Accordingly, hazardous substances, bad smell, or the like in the fluid can be efficiently decomposed/removed by the catalytic action of the photocatalyst. In addition, since the flowing direction of the fluid passing through the fluid passage path changes to the above-mentioned orthogonal direction, and the fluid temporarily stays between the rotating vane plate parts 41, the chances of contact between the fluid and the surfaces of the vane plate parts 41 carrying the photocatalyst are increased, whereby the above-described catalytic action can be enhanced. Since the fluid leading-out part is provided in a direction orthogonal to the fluid leading-in part as described above, variation in design can be increased. In the photocatalyst unit 15 according to the fifth embodiment as well, a generator that generates power in accordance with rotation of the impeller 4A and supply the power to the light application part 4 may be provided.

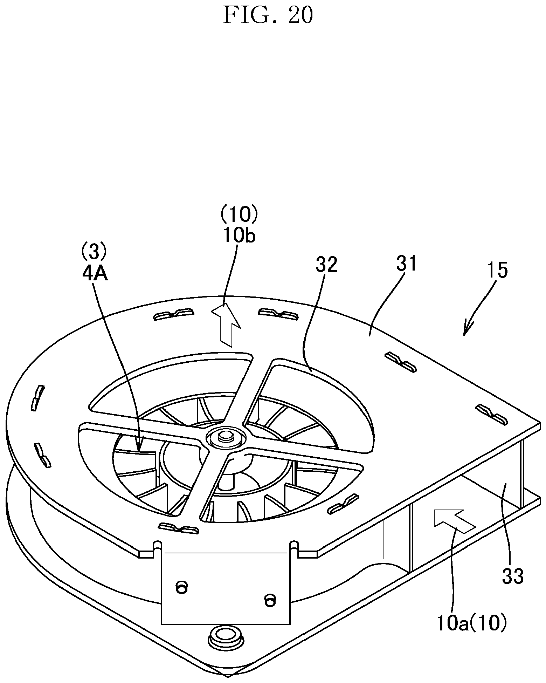

In the photocatalyst unit 15 according to the fifth embodiment, the leading-in direction of the fluid that is led in through the fluid leading-in part 10a is set to a direction along the axis of the impeller 4A, and the leading-out direction of the fluid that is led out through the fluid leading-out part 10b is set to a direction orthogonal to the axis of the impeller 4A. Here, the flow of the fluid may be reversed. Specifically, as shown in FIG. 20, the fluid passage path 10 may be configured by: a fluid leading-in part 10a that leads in a fluid through the opening portion 33 formed in the lateral surface of the box-shaped frame 31, into a direction orthogonal to the axis of the impeller 4A; and a fluid leading-out part 10b that leads out the fluid through the opening portion 32 formed in the upper surface of the box-shaped frame 31, toward a direction along the axis of the impeller 4A. Then, the impeller 4A is rotated in an opposite direction to that in the above-described case, whereby the fluid led in through the opening portion 33 on the lateral side from the direction orthogonal to the axis of the impeller 4A may be led out through the opening portion 32 on the upper side toward the direction along the axis of the impeller 4A.

### (Sixth embodiment)

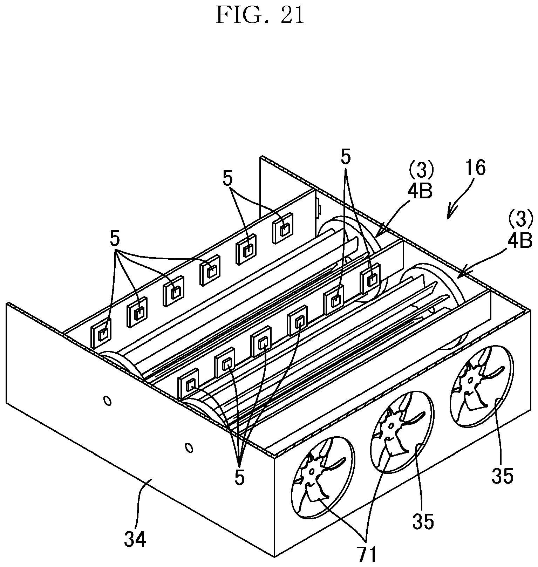

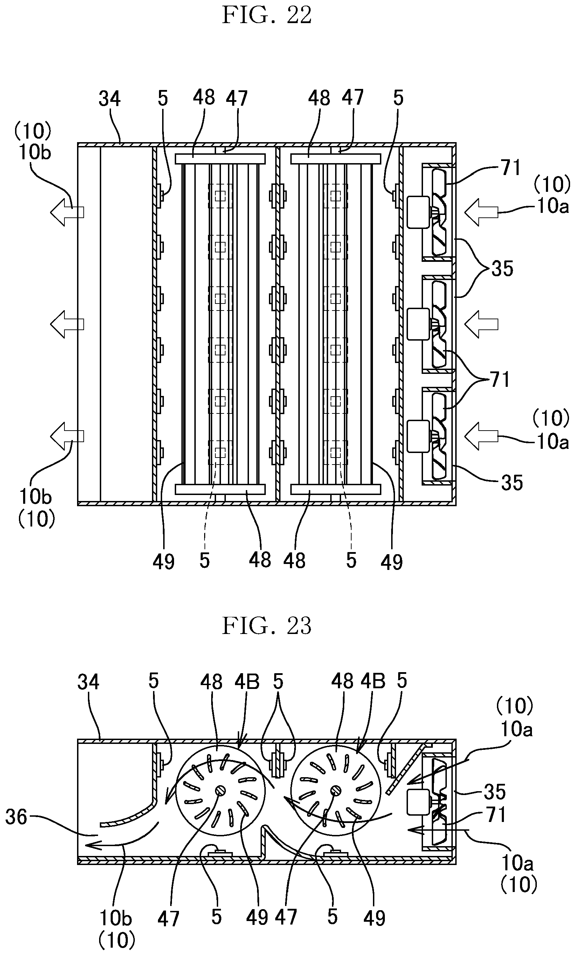

FIG. 21 to FIG. 23 show a photocatalyst unit 16 according to a sixth embodiment of the present invention. This photocatalyst unit 16 is configured such that two photocatalyst carriers 3 each implemented as a cross flow fan-like impeller 4B are provided in a box-shaped frame 34. The impeller 4B has a shape that is substantially similar to that of the impeller of a cross flow fan (also referred to as a tangential fan) which discharges a fluid in a manner of allowing the fluid to cross a shaft part 47 by a centrifugal force. Specifically, the impeller 4B includes: the shaft part 47 serving as the center of rotation thereof; a pair of end plates 48 fixed to the shaft part 47; and a plurality of vane plate parts 49 which carry a photocatalyst and are provided on the outer circumferential side of the shaft part 47 in a state where side end portions of the vane plate parts 49 are fixed to both end plates 48. Even when the wind pressure of the fluid flowing in a direction orthogonal to the axis of the impeller 4B is weak, the impeller 4B rotates in accordance with the wind pressure, to lead out the fluid toward the downstream side in the direction orthogonal to the axis of the impeller 4B.

The box-shaped frame 31 is provided with: three electric fans 71 installed so as to be respectively opposed to opening portions 35 formed in a front face of the box-shaped frame 31; and a plurality of light application parts 5 which each apply light from a predetermined position on the outer circumferential side with respect to the vane plate parts 49 of each impeller 4B, toward the rotating vane plate parts 49. Further, the box-shaped frame 34 has provided therein a fluid passage path 10 that includes: a fluid leading-in part 10a that leads a fluid into a direction orthogonal to the axis of the impeller 4B through an opening portion 35 in accordance with the blowing action of each electric fan 71; and a fluid leading-out part 10b that leads out the fluid through an opening portion 36 formed in the rear face of the box-shaped frame 31, to the downstream side of the direction orthogonal to the axis.

According to this configuration, in a state where light is being applied toward the rotating vane plate parts 41 from the light application parts 5, the fluid passing through the fluid passage path 10 is brought into contact with the photocatalyst carried by the surfaces of the vane plate parts 49 and the like. Accordingly, hazardous substances, bad smell, or the like in the fluid can be efficiently decomposed/removed by the catalytic action of the photocatalyst. In addition, since the time in which the fluid temporarily stays between the rotating vane plate parts 49 can be made longer than that of the blower fan-like impeller, the chances of contact between the fluid and the surfaces of the vane plate parts 49 carrying the photocatalyst are more increased, whereby the above-mentioned catalytic action can be enhanced. Since the dimension in the axis direction of the vane plate part 49 can be freely set, the fluid passage path 10 can be made large in the axis direction. Thus, the impeller 4B can be effectively rotated even when the wind pressure of the fluid passing through the fluid passage path 10 is weak, and can be efficiently installed also in a relatively large flow path. Thus, it is also easy to suppress the circulation resistance to be lower.

In the photocatalyst unit 16 according to the sixth embodiment as well, a generator that generates power in accordance with rotation of the impeller 4B and supplies the power to the light application part 5 may be provided. The number of photocatalyst carriers 3 each implemented as the cross flow fan-like impeller 4B provided in the box-shaped frame 34 is not limited to two, and may be one, three, or more.

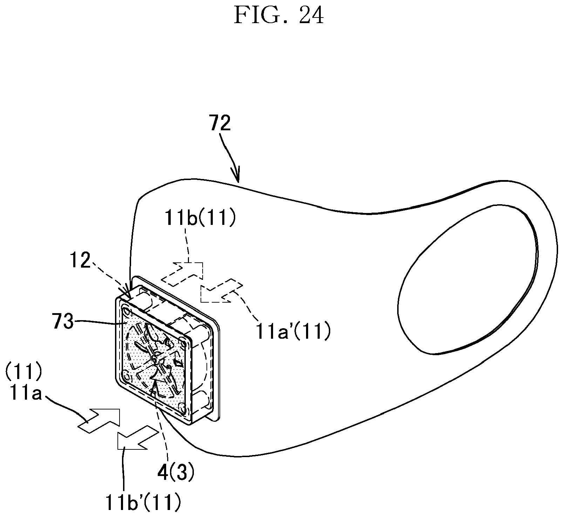

Next, an embodiment of a mask according to the present invention is described with reference to FIG. 24. A mask 72 according to the present embodiment is a sanitary mask such as a cloth mask or a non-woven fabric mask, in which the photocatalyst unit 12 (see FIG. 10 to FIG. 12) according to the second embodiment and including the photocatalyst carrier 3 implemented as a propeller fan-like impeller 4 is provided at an air passage part 11 caused in accordance with breathing of a mask wearer, specifically, at a position on the front side of the nose and the mouth of the mask wearer. The photocatalyst unit 12 is provided with a filtration filter 73 which covers a front side portion of the photocatalyst unit 12; and a light application part (not shown) which applies light to the vane plate parts of the impeller 4. The mask 72 is provided with a button battery which supplies power to the light application part, a jack which takes in power from an external power supply, a generator which supplies power generated in accordance with rotation of the impeller 4 to the light application part, or the like.

When the wearer of the mask 72 breathes in, air is led into the installation portion of the photocatalyst unit 12 from an air leading-in part 11a on the mask front side, whereby the impeller 4 rotates. Meanwhile, when the wearer of the mask 72 breathes out, air is led into the installation portion of the photocatalyst unit 12 from an air leading-in part 11a' on the mask rear side, whereby the impeller 4 rotates. Then, in a state where light is being applied to the vane plate parts of the impeller 4 from the light application part, air is brought into contact with the photocatalyst carried by the surfaces of the vane plate parts. Accordingly, hazardous components, bad smell components, or the like in the air can be efficiently decomposed/removed by the catalytic action of the photocatalyst, and viruses or the like in the air can be inactivated. Thereafter, when the wearer of the mask 72 breathes in, air is led out from an air leading-out part 11b on the mask rear side, and thus, viruses or the like can be prevented from entering the mouth or the like of the mask wearer. Meanwhile, when the wearer of the mask 72 breathes out, air is led out from an air leading-out part 11b' on the mask front side, and thus, viruses or the like can be prevented from being diffused into the atmosphere. In the present example, the photocatalyst unit is assembled to a sanitary mask, the entirety of which is made of a cloth, a non-woven fabric, or the like. However, the mask of the present invention is by no means limited thereto. A mask obtained by incorporating the photocatalyst unit according to the present invention into the part of a filter unit, of a dust prevention mask, which is assembled in a replaceable manner to a body part produced by use of a synthetic rubber or a synthetic resin; a mask obtained by mounting the photocatalyst unit and a filtration filter, in place of the part of the filter unit, to the dust prevention mask; and the like are also preferable embodiments.

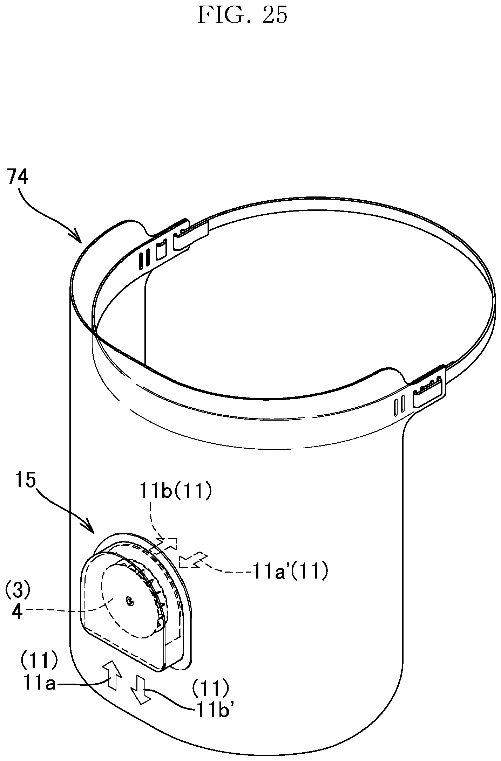

As shown in FIG. 25, instead of the embodiments described above, a droplet infection prevention mask 74 implemented as a so-called face shield formed by a transparent plastic plate or the like may be configured such that, on the back side thereof, the photocatalyst unit 15 of the fifth embodiment shown in FIG. 17 to FIG. 20, for example, is provided in an air passage part 11 that is caused in accordance with breathing of the mask wearer. Then, for example, when the mask wearer breathes in, air may be led in from an air leading-in part 11a positioned on the lower side or the like of the photocatalyst unit 15, and when the mask wearer breathes out, air may be led out from an air leading-out part 11b' positioned on the lower side or the like of the photocatalyst unit 15. The mask may not necessarily be a face shield, and may be a mask used as a mouth shield.

Then, in a state where light is being applied toward the rotating vane plate parts from the light application part (not shown), air is brought into contact with the photocatalyst carried by the surfaces of the vane plate parts. Accordingly, hazardous substances, bad smell, or the like in the air can be efficiently decomposed/removed by the catalytic action of the photocatalyst, and viruses or the like can be effectively inactivated. Thereafter, air is led out from the air leading-out part 11b on the mask rear side or the air leading-out part 11b' on the mask lower side. Accordingly, viruses or the like can be effectively prevented from entering the human body or viruses or the like can be effectively prevented from being diffused into the air.

Further, the photocatalyst unit shown in FIG. 17 and other drawings may be provided in an air passage part of a sanitary mask implemented as a cloth mask, a non-woven fabric mask, or the like, so that when the mask wearer breathes in, air is led into the installation portion of the photocatalyst unit from an air leading-in part on the mask lower side, and when the mask wearer breathes out, air is led into the installation portion of the photocatalyst unit from an air leading-in part on the mask rear side, whereby the impeller provided therein is caused to rotate.

While embodiments of the present invention have been described above, the present invention is by no means limited to these embodiments, and can be implemented, as a matter of course, in various forms without departing from the gist of the present invention. For example, in each embodiment, the support structure of the shaft of the impeller may be configured to be removable, thereby improving maintenance such as cleaning dirt attached to the vane plate parts or surroundings. Such a configuration is also a preferable embodiment.

### DESCRIPTION OF THE REFERENCE CHARACTERS

1, 1A, 12to 17 photocatalyst unit
2 photocatalyst filter
3 photocatalyst carrier
4, 4A, 4B impeller
5 light application part
6, 80, 81 reflection member
7, 70, 71 electric fan
8 ventilation duct
9 generator
10 fluid passage path
10a fluid leading-in part
10b fluid leading-out part
11 air passage part
11a air leading-in part
11b air leading-out part
20 lattice-shaped frame
21A cross part
21B cross part
21C standing piece
22 frame body
23 upper lid
30 cylindrical frame
40, 40A, 44, 45, 47 shaft part
41, 41A, 46, 49 vane plate part
42 bearing
50 substrate
51 light-emitting element
60 reflection surface
72, 74 mask
220, 221, 222, 223 side plate part

## Claims

1. A photocatalyst unit comprising:
a photocatalyst filter including a fluid passage path through which a fluid passes, and a photocatalyst body carrier, in the fluid passage path, having a surface that comes into contact with the fluid and carries a photocatalyst; and
a light application part that is provided inside the photocatalyst filter, and applies light composed of ultraviolet light or visible light toward the surface of the photocatalyst carrier, the surface carrying the photocatalyst, wherein
the photocatalyst carrier is an impeller having a plurality of vane plate parts each of which has a surface carrying the photocatalyst, the plurality of vane plate parts rotating about an axis upon receiving a pressure from the fluid passing through the fluid passage path, and
the light application part is provided to apply the light to the rotating vane plate parts of the impeller, from a predetermined position on an outer circumferential side with respect to the vane plate parts of the impeller.

2. The photocatalyst unit according to claim 1, wherein
the photocatalyst carrier is a propeller fan-like impeller that has: a shaft part that rotates about the axis; and a plurality of vane plate parts that are provided at an outer circumferential portion of the shaft part, each of the plurality of vane plate parts having a surface that carries the photocatalyst, and
the fluid passage path has a fluid leading-in part that leads in the fluid from a direction along the axis, and a fluid leading-out part that leads out the fluid toward a downstream side in the direction along the axis.

3. The photocatalyst unit according to claim 1 or 2, wherein
the photocatalyst filter has: a frame that is formed in a flat box shape having a small thickness relative to a size of each of upper and lower surfaces of the photocatalyst filter, and includes therein the fluid passage path; and a plurality of the impellers provided in parallel in a direction orthogonal to a thickness direction of the frame, and
one or a plurality of the light application parts are provided for each impeller.

4. The photocatalyst unit according to claim 3, wherein
the frame is a lattice-shaped frame having an upper surface and a lower surface, each of the upper surface and the lower surface having a gap through which a fluid passes,
the impeller is provided so as to stand in a rotatable manner at a predetermined cross part provided in the lattice-shaped frame, and
the light application part that applies light to the vane plate parts of the impeller is attached to another cross part in a surrounding of the cross part.

5. The photocatalyst unit according to claim 4, wherein
a standing piece is provided to the other cross part, and the light application part is fixed to the standing piece.

6. The photocatalyst unit according to any one of claims 3 to 5, wherein
the frame is provided with a rolling bearing that supports the impeller in a rotatable manner.

7. The photocatalyst unit according to claim 1 or 2, wherein
the photocatalyst filter has: a cylindrical frame extending in a direction along the axis; and a plurality of the impellers arrayed on a shaft part extending in a length direction of the cylindrical frame, and
one or a plurality of the light application parts are provided for each of the impellers.

8. The photocatalyst unit according to claim 1, wherein
the photocatalyst carrier is a blower fan-like impeller that has: a shaft part that rotates about the axis; and a plurality of vane plate parts that are provided to an outer circumferential side of the shaft part, each of the plurality of vane plate parts having a surface that carries the photocatalyst, and
the fluid passage path has a fluid leading-in part that leads in the fluid from a direction along the axis, or from one side in a direction orthogonal to the axis, and a fluid leading-out part that leads out the fluid in the direction along the axis, or toward another side in the direction orthogonal to the axis.

9. The photocatalyst unit according to claim 1, wherein
the photocatalyst carrier is a cross flow fan-like impeller that has: a shaft part that rotates about the axis; and a plurality of vane plate parts that are provided on an outer circumferential side of the shaft part, each of the plurality of vane plate parts having a surface that carries the photocatalyst, and
the fluid passage path has a fluid leading-in part that leads in the fluid from a direction orthogonal to the axis, and a fluid leading-out part that leads out the fluid from a position in the direction orthogonal to the axis, the position being different from a position of the fluid leading-in part.

10. The photocatalyst unit according to any one of claims 1 to 9, wherein
the light application part applies the light toward the axis of the impeller from the predetermined position.

11. The photocatalyst unit according to any one of claims 1 to 10, comprising
a reflection member that is provided on an outer circumferential side of the impeller, extends in a circumferential direction of the impeller, and has an inner circumferential surface that faces the impeller and serves as a reflection surface which reflects light.

12. The photocatalyst unit according to any one of claims 1 to 11, further comprising
a generator that generates power in accordance with rotation of the impeller, wherein
the light application part applies the light, using the power generated by the generator.

13. A mask comprising
the photocatalyst unit according to any one of claim 1, 2, or 8, wherein
the photocatalyst unit is provided in an air passage part that is generated in accordance with breathing of a mask wearer.
